# EUROPEAN PATENT APPLICATION

(11) **EP 3 922 108 A1**
(43) Date of publication of application: **15.12.2021**
(21) Application number: 20897340.4
(22) Date of filing: 26.09.2020
(51) Int. Cl.: A23L 27/00

(54) **METHOD FOR IMPROVING SENSORY ATTRIBUTE OF SUGAR ALCOHOL SUBSTANCE, AND APPLICATION THEREOF**

(30) Priority: 06.12.2019 CN 201911245282
(71) Applicant: Zhejiang Huakang Pharmaceutical Co., Ltd., Quzhou, Zhejiang 324302 (CN)
(72) Inventor: ZHANG, Wenyao, Quzhou, Zhejiang 324302 (CN); CHEN, Deshui, Quzhou, Zhejiang 324302 (CN); SHI, Lihua, Quzhou, Zhejiang 324302 (CN); ZUO, Qile, Quzhou, Zhejiang 324302 (CN); LI, Mian, Santa Clara, California 95054 (US); TANG, Wenjuan, Quzhou, Zhejiang 324302 (CN); TIAN, Shiyi, Quzhou, Zhejiang 324302 (CN)
(74) Representative: Sun, Yiming
(86) International application number: PCT/CN2020/117962
(87) International publication number: WO 2021/109692

(57) **Abstract**

The present invention relates to a method for improving sensory attributes of a sugar alcohol and application thereof. After analyzing the difference between the sugar alcohol used as a sugar substitute and sucrose in multiple sensory attribute dimensions by flavor analysis, corresponding taste modifying substances are added according to the intensity of the difference, thereby solving the problem that there are differences between the sugar alcohol used as a sugar substitute and the sucrose in taste sensory attributes in the field of food at present. According to the invention, the taste difference between the sugar alcohol and the sucrose in multiple sensory attribute dimensions is analyzed, so that the level of taste improvement is broader. Multidimensional accurate quantification of differences is realized by means of a sensory flavor wheel, thereby realizing accurate taste modification and improving the accuracy of taste improvement.

## Description

### TECHNICAL FIELD

The present invention belongs to the technical field of sensory measurement for foods, particularly relates to a method for improving sensory attributes of a sugar alcohol and application thereof.

### BACKGROUND

As we all know, there are multiple taste attributes that can be sensed by humans, such as sweetness attribute, sourness attribute, bitterness attribute, saltiness attribute, umami attribute, spiciness attribute, numbness attribute and the like. Correspondingly, there are multiple taste substances in each taste attribute that can give people the same taste sensation, for example, sucrose, fructose and xylitol in the sweetness attribute, citric acid, malic acid, tartaric acid in the sourness attribute, and the like.

In addition, although different taste substances in the same taste attribute can present the same taste sensation, there still may be other different sensory attributes. For example, sucralose in the sweetness attribute has a metallic taste sensory attribute as compared with sucrose, and potassium chloride in the saltiness attribute has a bitterness sensory attribute as compared with sodium chloride. Therefore, in the field of foods, especially food research and development personnel need to take into account the influence of sensory attributes of various taste substances when various taste substances are added to actual products. As a result, a series of appropriate and accurate sensory evaluation attributes can not only fully reflect the true sensory characteristics of a single taste substance, but also help sensory tasters and scientific researchers to accurately distinguish and apply different taste substances.

At present, in the sensory field of foods, there are almost no technical methods to determine the sensory evaluation attributes of taste substances. The relevant staff in the field of foods basically determines the sensory evaluation attributes for evaluating taste substances based on their work experiences or with reference to the relevant research work of others. However, although this experience-based sensory evaluation attribute determination method can reflect the sensory characteristics of the taste substances to some extent, the true correlation and validity of the taste attributes and sensory characteristics determined by this method are not clear from a certain perspective because statistic analysis is not performed.

### SUMMARY

The technical problem to be solved by the present invention is to provide a method for improving sensory attributes of a sugar alcohol and application thereof. After analyzing the difference between the sugar alcohol used as a sugar substitute and sucrose in multiple sensory attribute dimensions, corresponding taste modifying substances are added according to the intensity of the difference, thereby solving the problem that the sugar alcohol used as a sugar substitute and the sucrose are different in taste sensory attributes in the field of food at present.

The present invention is realized by providing a method for improving sensory attributes of a sugar alcohol, including the following steps:
step I, respectively performing, by a plurality of sensory tasters, intensity evaluation of a total of six sensory attribute dimensions, i.e. sweetness, after-sweet taste, bitterness, astringency, metallic taste and salivation, for a sugar alcohol to be evaluated and sucrose both having a same concentration by using a nine-point scale, and recording evaluation results respectively;
step II, respectively calculating average values of the intensity evaluation results of the six sensory attribute dimensions of the sugar alcohol to be evaluated and the sucrose in step I, and obtaining taste difference values between the sugar alcohol to be evaluated and the sucrose in the six sensory attribute dimensions by performing sensory flavor wheel analysis with the average values; and
step III, for the sensory attributes with the taste difference value being 1 or more intensity units in step II, respectively adding a corresponding taste modifying substance by the following manner to perform taste improvement:
   (1) if the sweetness sensory attribute of the sugar alcohol to be evaluated is lower than that of the sucrose by 1 or more intensity units, adding sodium chloride according to 1-10 mg/kg•intensity unit; if the sweetness sensory attribute of the sugar alcohol to be evaluated is higher than that of the sucrose by 1 intensity unit, adding a lemon peel extract according to 1-5 mg/kg•intensity unit;
   (2) if the after-sweet taste sensory attribute of the sugar alcohol to be evaluated is lower than that of the sucrose by 1 or more intensity units, adding sodium chloride according to 1-3 mg/kg•intensity unit and adding steviol glycoside according to 1-200 mg/kg•intensity unit; if the after-sweet taste sensory attribute of the sugar alcohol to be evaluated is higher than that of the sucrose by 1 intensity unit, adding a lemon peel extract according to 1-10 mg/kg•intensity unit;
   (3) if the bitterness sensory attribute of the sugar alcohol to be evaluated is lower than that of the sucrose by 1 or more intensity units, adding nothing; if the bitterness sensory attribute of the sugar alcohol to be evaluated is higher than that of the sucrose by 1 or more intensity units, adding L-asparagine according to 1-10 mg/kg•intensity unit;
   (4) if the astringency sensory attribute of the sugar alcohol to be evaluated is lower than that of the sucrose by 1 or more intensity units, adding nothing; if the astringency sensory attribute of the sugar alcohol to be evaluated is higher than that of the sucrose by 1 or more intensity units, adding arabinose according to 1-100 mg/kg•intensity unit;
   (5) if the metallic taste sensory attribute of the sugar alcohol to be evaluated is lower than that of the sucrose by 1 or more intensity units, adding nothing; if the metallic taste sensory attribute of the sugar alcohol to be evaluated is higher than that of the sucrose by 1 or more intensity units, adding maltose according to 1-800 mg/kg•intensity unit;
   (6) if the salivation sensory attribute of the sugar alcohol to be evaluated is lower than that of the sucrose by 1 or more intensity units, adding mannitol according to 1-250 mg/kg•intensity unit; and if the salivation sensory attribute of the sugar alcohol to be evaluated is higher than that of the sucrose by 1 or more intensity units, adding nothing.

Compared with the prior art, the method for improving sensory attributes of a sugar alcohol and the application thereof according to the present invention have the following characteristics:
1. By flavor analysis, the taste difference between the sugar alcohol used as a sugar substitute and the sucrose in multiple sensory attribute dimensions is analyzed, and then taste modification is performed, so that the level of taste improvement is broader and the accuracy is improved.
2. By means of a sensory flavor wheel analysis diagram, multidimensional accurate quantification of differences between the sugar alcohol used as a sugar substitute and the sucrose can be realized, thereby realizing accurate taste modification and higher accuracy of taste improvement.
3. The problem that there are differences between the sugar alcohol used as a sugar substitute and the sucrose in taste sensory attributes in the field of food at present is solved.

### BRIEF DESCRIPTION OF THE DRAWINGS

Fig. 1 is a sensory flavor wheel analysis result diagram of xylitol and sucrose drawn using a method for improving sensory attributes of a sugar alcohol according to the present invention.
Fig. 2 is a sensory flavor wheel analysis result diagram of erythritol and sucrose drawn using a method for improving sensory attributes of a sugar alcohol according to the present invention.

### DETAILED DESCRIPTION OF THE EMBODIMENTS

In order to make the technical problems to be solved, technical solutions and advantageous effects of the present invention clearer, the present invention will be described in detail below with reference to the accompanying drawing and examples. It should be understood that the specific examples described herein are merely illustrative of the present invention and are not intended to limit the present invention.

A preferred example of a method for improving sensory attributes of a sugar alcohol according to the present invention includes the following steps.

At step I, a plurality of sensory tasters respectively perform intensity evaluation of six sensory attribute dimensions, i.e. sweetness, after-sweet taste, bitterness, astringency, metallic taste and salivation, for a sugar alcohol to be evaluated and sucrose both having a same concentration by using a nine-point scale, and also respectively record evaluation results.

At step II, average values of the intensity evaluation results of the six sensory attribute dimensions of the sugar alcohol to be evaluated and the sucrose in step I are respectively calculated, sensory flavor wheel analysis is performed with the average values to draw a sensory flavor wheel analysis result diagram, so as to obtain taste difference values between the sugar alcohol to be evaluated and the sucrose in the six sensory attribute dimensions.

At step III, for the sensory attributes with the taste difference value being 1 or more intensity units in step II, a corresponding taste modifying substance is respectively added by the following manner to perform taste improvement.
(1) If the sweetness sensory attribute of the sugar alcohol to be evaluated is lower than that of the sucrose by 1 or more intensity units, sodium chloride is added according to 1-10 mg/kg•intensity unit. If the sweetness sensory attribute of the sugar alcohol to be evaluated is higher than that of the sucrose by 1 intensity unit, a lemon peel extract is added according to 1-5 mg/kg•intensity unit.
(2) If the after-sweet taste sensory attribute of the sugar alcohol to be evaluated is lower than that of the sucrose by 1 or more intensity units, sodium chloride is added according to 1-3 mg/kg•intensity unit and steviol glycoside is added according to 1-200 mg/kg•intensity unit. If the after-sweet taste sensory attribute of the sugar alcohol to be evaluated is higher than that of the sucrose by 1 intensity unit, a lemon peel extract is added according to 1-10 mg/kg•intensity unit.
(3) If the bitterness sensory attribute of the sugar alcohol to be evaluated is lower than that of the sucrose by 1 or more intensity units, nothing is added. If the bitterness sensory attribute of the sugar alcohol to be evaluated is higher than that of the sucrose by 1 or more intensity units, L-asparagine is added according to 1-10 mg/kg•intensity unit.
(4) If the astringency sensory attribute of the sugar alcohol to be evaluated is lower than that of the sucrose by 1 or more intensity units, nothing is added. If the astringency sensory attribute of the sugar alcohol to be evaluated is higher than that of the sucrose by 1 or more intensity units, arabinose is added according to 1-100 mg/kg•intensity unit.
(5) If the metallic taste sensory attribute of the sugar alcohol to be evaluated is lower than that of the sucrose by 1 or more intensity units, nothing is added. If the metallic taste sensory attribute of the sugar alcohol to be evaluated is higher than that of the sucrose by 1 or more intensity units, maltose is added according to 1-800 mg/kg•intensity unit.
(6) If the salivation sensory attribute of the sugar alcohol to be evaluated is lower than that of the sucrose by 1 or more intensity units, mannitol is added according to 1-250 mg/kg•intensity unit. If the salivation sensory attribute of the sugar alcohol to be evaluated is higher than that of the sucrose by 1 or more intensity units, nothing is added.

Specifically, in step I, the nine-point scale uses numbers 1-9 to represent 9 intensity units, respectively corresponding to dislike extremely, dislike very much, dislike moderately, dislike slightly, neither like nor dislike, like slightly, like moderately, like very much and like extremely. There are at least 10 sensory tasters. The sensory tasters may be differentiated by gender and age group according to the evaluation needs.

Specifically, the sugar alcohol to be evaluated is any one of xylitol, erythritol, sorbitol, maltitol, isomalt, lactitol, tagatose, polyols, sucralose, steviol glycoside and acesulfame potassium.

The method of the present invention will be further described below in conjunction with specific examples.

### Example 1

In a first preferred example of the method for improving sensory attributes of a sugar alcohol according to the present invention, the sugar alcohol to be evaluated was xylitol, and the method specifically included the following steps:

Step I, 30 sensory tasters respectively performed intensity evaluation of six sensory attribute dimensions, i.e., sweetness, after-sweet taste, bitterness, astringency, metallic taste and salivation, for the xylitol and sucrose both having a content of 5% by using a nine-point scale.

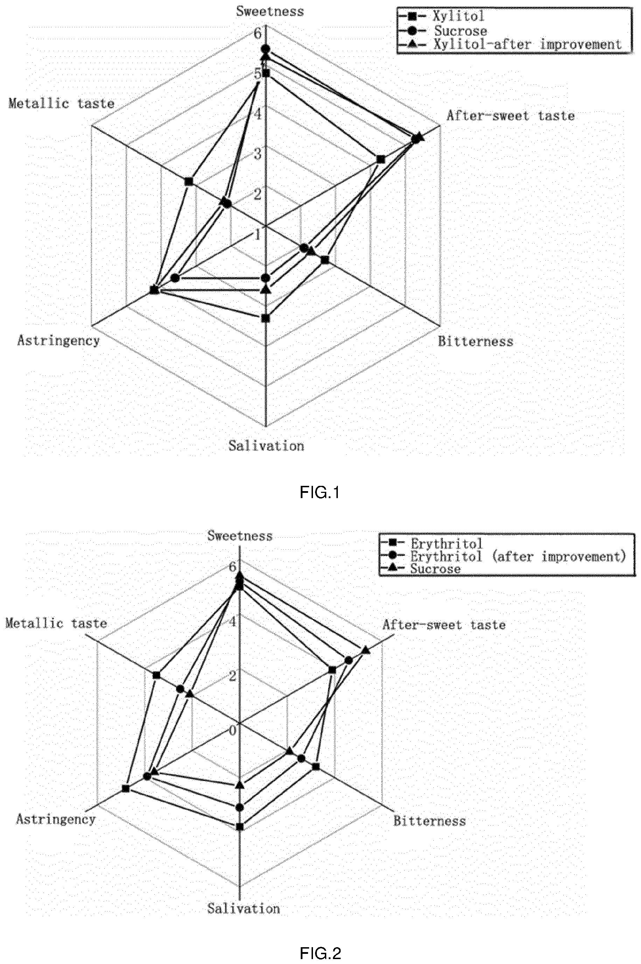

Step II, average values of the intensity evaluation results of the six sensory attribute dimensions of the xylitol and the sucrose in step I were respectively calculated, and sensory flavor wheel analysis was performed with the average values to obtain taste difference values between the xylitol and the sucrose in the six sensory attribute dimensions. The results are shown in the following table. A sensory flavor wheel analysis result diagram was drawn, as shown in Fig. 1.

**[Table 1]**

| Attribute dimension | Xylitol | Sucrose |
|---|---|---|
| Sweetness | 4.8 | 5.4 |
| After-sweet taste | 4.3 | 5.3 |
| Bitterness | 2.7 | 2.1 |
| Salivation | 3.3 | 2.3 |
| Astringency | 4.2 | 3.6 |
| Metallic taste | 3.2 | 2.1 |

Step III, for the sensory attributes with the taste difference value being 1 or more intensity units in step II, a corresponding taste modifying substance was respectively added to perform taste improvement. The comparison results of the values before and after the improvement are shown in the following table.

**[Table 2]**

| Attribute dimension | Xylitol (before improvement) | Xylitol (after improvement) | Sucrose |
|---|---|---|---|
| Sweetness | 4.8 | 5.2 | 5.4 |
| After-Sweet taste | 4.3 | 5.4 | 5.3 |
| Bitterness | 2.7 | 2.3 | 2.1 |
| Salivation | 3.3 | 2.6 | 2.3 |
| Astringency | 4.2 | 4.2 | 3.6 |
| Metallic taste | 3.2 | 2.2 | 2.1 |

It can be seen from the table above that after the taste modifying substance was added to the xylitol with the content of 5%, the taste sensory attribute difference between the xylitol and the sucrose was significantly reduced.

### Example 2

In a second preferred example of the method for improving sensory attributes of a sugar alcohol according to the invention, the sugar alcohol to be evaluated was erythritol, the method specifically included the following steps:

Step I, 30 sensory tasters respectively performed intensity evaluation of six sensory attribute dimensions, i.e. sweetness, after-sweet taste, bitterness, astringency, metallic taste and salivation, for the erythritol and sucrose both having a content of 8% by using a nine-point scale.

Step II, average values of the intensity evaluation of the six sensory attribute dimensions of the erythritol and the sucrose in step II were respectively calculated, and sensory flavor wheel analysis was performed with the average values to obtain taste difference values between the erythritol and the sucrose in the six sensory attribute dimensions. The results are shown in the following table. A sensory flavor wheel analysis result diagram was drawn, as shown in Fig. 2.

**[Table 3]**

| Attribute dimension | Erythritol | Sucrose |
|---|---|---|
| Sweetness | 5.0 | 5.4 |
| After-sweet taste | 3.9 | 5.3 |
| Bitterness | 3.2 | 2.1 |
| Salivation | 3.8 | 2.3 |
| Astringency | 4.8 | 3.6 |
| Metallic taste | 3.5 | 2.1 |

Step III, for the sensory attributes with the taste difference value being 1 or more intensity units in step II, a corresponding taste modifying substance was respectively added to perform taste improvement. The comparison results of the values before and after the improvement are shown in the following table.

**[Table 4]**

| Attribute dimension | Erythritol (before improvement) | Erythritol (after improvement) | Sucrose |
|---|---|---|---|
| Sweetness | 5.0 | 5.2 | 5.4 |
| After-sweet taste | 3.9 | 4.6 | 5.3 |
| Bitterness | 3.2 | 2.6 | 2.1 |
| Salivation | 3.8 | 3.1 | 2.3 |
| Astringency | 4.8 | 3.9 | 3.6 |
| Metallic taste | 3.5 | 2.5 | 2.1 |

It can be seen from the table above that after the taste modifying substance was added to the erythritol with the content of 8%, the taste sensory attribute difference between the erythritol and the sucrose was significantly reduced.

The present invention further discloses an application of the method for improving sensory attributes of a sugar alcohol as described above. The application includes improving the flavor of a sweetener, or improving the flavor of a souring agent, or improving the flavor of a bittering agent. In application, the sucrose in the method of the present invention is replaced with other sweeteners or souring agents or bittering agents, and the method of the present invention can be used for similar flavor adjustment and improvement.

The above description is only the preferred examples of the invention and is not intended to limit the invention. Any modifications, equivalent substitutions and improvements made within the spirit and scope of the invention should be included within the protection scope of the invention.

## Claims

1. A method for improving sensory attributes of a sugar alcohol, comprising the following steps:
at step I, respectively performing, by a plurality of sensory tasters, intensity evaluation of six sensory attribute dimensions, i.e. sweetness, after-sweet taste, bitterness, astringency, metallic taste and salivation, for a sugar alcohol to be evaluated and sucrose both having a same concentration by using a nine-point scale, and respectively recording evaluation results;
at step II, respectively calculating average values of the intensity evaluation results of the six sensory attribute dimensions of the sugar alcohol to be evaluated and the sucrose in step I, and performing sensory flavor wheel analysis with the average values to obtain taste difference values between the sugar alcohol to be evaluated and the sucrose in the six sensory attribute dimensions; and
at step III, for the sensory attributes with the taste difference value being 1 or more intensity units in step II, respectively adding a corresponding taste modifying substance by the following manner to perform taste improvement:
(1) if the sweetness sensory attribute of the sugar alcohol to be evaluated is lower than that of the sucrose by 1 or more intensity units, adding sodium chloride according to 1-10 mg/kg•intensity unit; if the sweetness sensory attribute of the sugar alcohol to be evaluated is higher than that of the sucrose by 1 intensity unit, adding a lemon peel extract according to 1-5 mg/kg•intensity unit;
(2) if the after-sweet taste sensory attribute of the sugar alcohol to be evaluated is lower than that of the sucrose by 1 or more intensity units, adding sodium chloride according to 1-3 mg/kg•intensity unit and adding steviol glycoside according to 1-200 mg/kg•intensity unit; if the after-sweet taste sensory attribute of the sugar alcohol to be evaluated is higher than that of the sucrose by 1 intensity unit, adding a lemon peel extract according to 1-10 mg/kg•intensity unit;
(3) if the bitterness sensory attribute of the sugar alcohol to be evaluated is lower than that of the sucrose by 1 or more intensity units, adding nothing; if the bitterness sensory attribute of the sugar alcohol to be evaluated is higher than that of the sucrose by 1 or more intensity units, adding L-asparagine according to 1-10 mg/kg•intensity unit;
(4) if the astringency sensory attribute of the sugar alcohol to be evaluated is lower than that of the sucrose by 1 or more intensity units, adding nothing; if the astringency sensory attribute of the sugar alcohol to be evaluated is higher than that of the sucrose by 1 or more intensity units, adding arabinose according to 1-100 mg/kg•intensity unit;
(5) if the metallic taste sensory attribute of the sugar alcohol to be evaluated is lower than that of the sucrose by 1 or more intensity units, adding nothing; if the metallic taste sensory attribute of the sugar alcohol to be evaluated is higher than that of the sucrose by 1 or more intensity units, adding maltose according to 1-800 mg/kg•intensity unit;
(6) if the salivation sensory attribute of the sugar alcohol to be evaluated is lower than that of the sucrose by 1 or more intensity units, adding mannitol according to 1-250 mg/kg•intensity unit; and if the salivation sensory attribute of the sugar alcohol to be evaluated is higher than that of the sucrose by 1 or more intensity units, adding nothing.

2. The method for improving sensory attributes of a sugar alcohol according to claim 1, wherein the nine-point scale in step I uses numbers 1-9 to represent 9 intensity units, respectively corresponding to dislike extremely, dislike very much, dislike moderately, dislike slightly, neither like nor dislike, like slightly, like moderately, like very much and like extremely.

3. The method for improving sensory attributes of a sugar alcohol according to claim 1, wherein the number of the sensory tasters in step I is at least 10.

4. The method for improving sensory attributes of a sugar alcohol according to claim 1, wherein the sugar alcohol to be evaluated is any one of xylitol, erythritol, sorbitol, maltitol, isomalt, lactitol, tagatose, polyols, sucralose, steviol glycoside and acesulfame potassium.

5. An application of the method for improving sensory attributes of a sugar alcohol according to any of claims 1 to 4, wherein the application comprises improving the flavor of a sweetener, or improving the flavor of a souring agent, or improving the flavor of a bittering agent.
